# EUROPÄISCHE PATENTANMELDUNG

(11) **EP 2 425 870 A1**
(43) Veröffentlichungstag der Anmeldung: **07.03.2012**
(21) Anmeldenummer: 11005238.8
(22) Anmeldetag: 28.06.2011
(51) Int. Cl.: A61M 39/08, A61M 25/00

(54) **Mehr-Lumen-Verlängerung**

(30) Priorität: 05.07.2010 CH 10932010
(71) Anmelder: Schmidlin, Daniel, 8006 Zürich (CH)
(72) Erfinder: Schmidlin, Daniel, 8006 Zürich (CH)
(74) Vertreter: Schaad, Balass, Menzl & Partner AG

(57) **Zusammenfassung**

Die Mehr-Lumen-Verlängerung umfasst mindestens zwei longitudinale, unterschiedliche Farbcodierungen, einen Innenmantel (11) mit einem wenigstens annährend kreisrunden Querschnitt und einen Aussenmantel (11') mit einem wenigstens annährend kreisrunden Querschnitt aufweisende Einzel-Lumen-Verlängerungen (12), welche je einen an ein distales Ende (14) anschliessenden distalen Endbereich (16) und je einen an ein proximales Ende (18) anschliessenden proximalen Endbereich (20) aufweisen. Das proximale Ende (18) der Mehr-Lumen-Verlängerung (10) ist dazu bestimmt, an einen Katheter, und das distale Ende (14) der Mehr-Lumen-Verlängerung (10) ist dazu bestimmt, an eine medizinische Quelle angeschlossen zu werden. Dem distalen Ende (14) ist dazu ein distales Kupplungselement (22) und dem proximalen Ende (18) ein proximales Kupplungselement (24) zugeordnet. Erfindungsgemäss sind die Einzel-Lumen-Verlängerungen (12) in einem zwischen dem distalen Endbereich (16) und dem proximalen Endbereich (20) liegenden Zwischenbereich (26) nebeneinander angeordnet und mindestens abschnittsweise aneinander befestigt. Erfindungsgemäss sind die Einzel-Lumen-Verlängerungen (12) in den beiden Endbereichen (16, 20) voneinander getrennt.

## Beschreibung

Die vorliegende Erfindung betrifft eine Mehr-Lumen-Verlängerung gemäss Anspruch 1 und ein Verfahren zur Herstellung der Mehr-Lumen-Verlängerung gemäss Anspruch 13.

In der Medizin und der Biologie wird der Begriff Lumen verwendet, um den Hohlraum eines röhrenförmigen Körperorgans wie beispielsweise eines Blutgefässes zu definieren. Verbindungselemente, welche entsprechend das Blutgefäss mit einer medizinischen Quelle verbinden, können somit als Lumen-Verlängerungen bezeichnet werden. Da eine solche Lumen-Verlängerung den Körperstamm mit einer medizinischen Quelle verbindet, kann ein proximales Ende zum Körperstamm zeigend und ein distales Ende zur Quelle zeigend, für die Lumen-Verlängerung definiert werden.

Lumen-Verlängerungen werden bevorzugt für medizinische Anwendungen, wie beispielsweise zur Zuführung von intravenösen Medikamenten auch in Anästhesie und Intensivmedizin, eingesetzt. Entsprechend können Lumen-Verlängerungen am proximalen Ende mit einem irgendwie gearteten Katheter verbunden sein, welcher als Verweilkatheter in einer Vene oder einer Arterie liegt. Am distalen Ende der Lumen-Verlängerung werden entsprechend die medizinischen Quellen, wie beispielsweise Infusionspumpen oder Infusionsbeutel mit einer Regelvorrichtung zur Dosierung der gewünschten Flüssigkeitsmenge, welche in das Blutgefäss des Patienten geführt werden soll, angeschlossen.

Handelsübliche Lumen-Verlängerungen können als Einzel-Lumen-Verlängerungen oder als Multi-Lumen-Verlängerungen ausgebildet sein.

Eine Mehr-Lumen-Verlängerung der vorliegenden Art ist aus Dokument US 2010/0168642 A1 bekannt. Die Mehr-Lumen-Verlängerung besteht aus genau zwei farbcodierten, einen Innenmantel und einen Aussenmantel aufweisenden Einzel-Lumen-Verlängerungen, wobei die beiden Aussenmäntel in einem Zwischenbereich miteinander fest verbunden sind. Dabei sind - im Querschnitt - die Innenmäntel sowie Aussenmäntel der beiden Einzel-Lumen-Verlängerungen halbkreisförmig und zwischen den Enden der Halbkreisform geradlinig geformt, so dass die beiden Aussenmäntel eine Berührungsfläche bilden und die halbkreisförmigen Einzel-Lumen-Verlängerungen den Durchmesser der kreiszylinderförmigen Mehr-Lumen-Verlängerung ausbilden.

Multi-Lumen-Verlängerungen bilden eine Untergruppe der Einzel-Lumen-Verlängerungen, wobei mindestens zwei Einzel-Lumen-Verlängerungen auf eine einzige Einzel-Lumen-Verlängerung geführt werden, um beispielsweise verschiedene medizinische Quellen auf einen Zugang zu führen, um den Einsatz von mehreren Zugängen am Patienten zu vermeiden, da dies durch das Stechen mit Schmerzen verbunden ist.

Eine mögliche Multi-Lumen-Verlängerung ist derart gestaltet, dass eine einzige Einzel-Lumen-Verlängerung im Innenraum der Lumen-Verlängerung mindestens zwei Lumen ausbildet, welche über eine Wand gleichen Materials wie der Mantel voneinander räumlich getrennt sind und so zwei unterschiedliche Einzel-Lumen-Verlängerungen ausbilden. Hierzu wird auf das Dokument WO 03/037415 A1 verwiesen, wo zwar ein Ballonkatheter offenbart wird, jedoch auch der Einsatz als Infusionskatheter möglich ist.

Eine weitere Multi-Lumen-Verlängerung wird in Dokument US 2002/0103066 A1 offenbart. Hierbei wird ebenfalls eine einzige Einzel-Lumen-Verlängerung im Innenraum mit mindestens zwei oder mehr Lumen versehen, wobei der einzige Aussenmantel entweder einen rechteckigen oder dreieckigen Querschnitt aufweisen kann.

Eine weitere Multi-Lumen-Verlängerung ist derart gestaltet, dass beispielsweise mindestens zwei separat verlaufende Einzel-Lumen-Verlängerungen über einen Verbindungsanschluss mit einer einzigen Einzel-Lumen-Verlängerung verbunden sind und die Anschlüsse am distalen Ende farblich codiert sein können. Hierzu wird beispielhaft auf das Dokument US 5,207,643 verwiesen.

Nachteilig an oben genannten Multi-Lumen-Verlängerungen, welche zur Untergruppe der Einzel-Lumen-Verlängerungen zählen, ist die Verwechslungsgefahr bei einem Einsatz einer Vielzahl dieser Verlängerungen in der Intensivmedizin. Gerade in der Intensivmedizin besteht die Aufgabe darin, eine Vielzahl von medizinischen Quellen mit einer Vielzahl von Zugängen zur gleichen Zeit im Einsatz zu haben, wo eine falsche Zuordnung verheerende Folgen für den Patienten hätte. Ein weiterer Nachteil in der Intensivmedizin ist die Gefahr, dass sich die Einzel-Lumen-Verlängerungen im Einsatz verheddern könnten und so eine eindeutige Zuordnung nur mit erheblichem Zeitaufwand gewährleistet ist, so dass auch ein entsprechendes Handling stark beeinträchtigt ist.

Um den Nachteil der Verwechslungsgefahr zu minimieren sind Infusionskatheter bekannt, bei denen der Mantel der Einzel-Lumen-Verlängerung beispielsweise eingefärbt, mit einem gefärbten Band umhüllt oder mit einer anderweitigen Markierung, wie Muster auf der Manteloberfläche, versehen wird. Beispielhalber wird diesbezüglich auf US-B-6059768 und US-A-20020058928 verwiesen.

Der Nachteil des Verhedderns von Einzel-Lumen-Verlängerungen beim Einsatz in der Intensivmedizin wird durch die farbliche Codierung jedoch nicht ausreichend gelöst.

Der vorliegenden Erfindung liegt daher die Aufgabe zugrunde, eine mindestens zwei Einzel-Lumen-Verlängerung umfassende Mehr-Lumen-Verlängerung bereitzustellen, welche eine eindeutige Zuordnung der jeweiligen Enden ermöglicht, eine einfache Handhabung gewährleistet und die Gefahr des Verhedderns der Einzel-Lumen-Verlängerungen minimiert.

Diese Aufgabe wird mit einer Mehr-Lumen-Verlängerung gemäss dem Anspruch 1 gelöst.

Eine Erfindungsgemässe Mehr-Lumen-Verlängerung umfasst mindestens zwei longitudinale, unterschiedlich gefärbte, je einen Innenmantel mit einem wenigstens annährend kreisrunden Querschnitt und je einen Aussenmantel mit einem wenigstens annährend kreisrunden Querschnitt aufweisende Einzel-Lumen-Verlängerungen, welche je einen an ein distales Ende anschliessenden distalen Endbereich und je einen an ein proximales Ende anschliessenden proximalen Endbereich aufweisen. Das proximale Ende der Mehr-Lumen-Verlängerung ist dazu bestimmt, an einen Katheter, und das distale Ende der Mehr-Lumen-Verlängerung ist dazu bestimmt, an eine medizinische Quelle angeschlossen zu werden. Dazu ist dem distalen Ende ein distales Kupplungselement und dem proximalen Ende ein proximales Kupplungselement zugeordnet. Erfindungsgemäss sind die Einzel-Lumen-Verlängerungen in einem zwischen dem distalen Endbereich und dem proximalen Endbereich liegenden Zwischenbereich nebeneinander angeordnet und mindestens abschnittsweise aneinander befestigt. Erfindungsgemäss sind des Weiteren die Einzel-Lumen-Verlängerungen in den beiden Endbereichen voneinander getrennt, dass heisst, nicht aneinander befestigt.

Eine erfindungsgemässe Mehr-Lumen-Verlängerung kann beispielsweise in der Intensivmedizin eingesetzt werden, da die Einzel-Lumen-Verlängerungen im Einsatz eine gerichtete Ordnung aufweisen und die Gefahr des Verhedderns erheblich minimiert ist, da die Einzel-Lumen-Verlängerungen abschnittsweise aneinander befestigt sind. Zudem sind die einzelnen Einzel-Lumen-Verlängerungen der Mehr-Lumen-Verlängerung eingefärbt, so dass auch eine Verwechslungsgefahr im Einsatz erheblich reduziert ist. Dadurch, dass im Endbereich die Mehr-Lumen-Verlängerungen getrennt sind, ergibt sich ein grosser Freiheitsgrad für das anschliessen der medizinischen Quelle am distalen Ende und das anschliessen des Zugangs am proximalen Ende, ohne dabei die gerichtete Ordnung zu verlieren.

Bei einer weiteren erfindungsgemäss bevorzugten Ausführungsform sind die Einzel-Lumen-Verlängerungen im Zwischenbereich ununterbrochen aneinander befestigt.

Bei einer weiteren erfindungsgemäss bevorzugten Ausführungsform ist die Befestigung zwischen den Einzel-Lumen-Verlängerungen vom distalen Endbereich und dem proximalen Endbereich her lösbar. Dies bewirkt, dass an die Enden der Einzel-Lumen-Verlängerungen die entsprechenden Kupplungselemente einfach montiert werden können. Somit können zwei benachbarte Einzel-Lumen-Verlängerungen im Endbereich aufgetrennt werden und bilden einen verlängerten Endbereich, wobei die benachbarten Einzel-Lumen-Verlängerungen unberührt bleiben. Dies wiederum bewirkt einen grossen Freiheitsgrad für die Einzel-Lumen-Verlängerungen im Endbereich und eine flexible Zuordnung im proximalen ebenso wie im distalen Endbereich für den Anschluss oder das Trennen von medizinischen Quellen und/oder Kathetern.

Bei einer weiteren erfindungsgemäss bevorzugten Ausführungsform sind die Einzel-Lumen-Verlängerungen im Zwischenbereich abschnittsweise verschweisst oder verklebt.

Bei einer weiteren erfindungsgemäss bevorzugten Ausführungsform bilden die Aussenmäntel von mindestens zwei benachbarten Einzel-Lumen-Verlängerungen im Zwischenbereich mindestens abschnittsweise einen linienförmigen Berührungsabschnitt und die Verschweissung oder Verklebung liegt in diesem linienförmigen Berührungsabschnitt. Dies ermöglicht eine flexible Anordnung von einer Vielzahl von zueinander benachbarten Einzel-Lumen-Verlängerungen zu einer erfindungsgemässen Mehr-Lumen-Verlängerung.

Bei einer weiteren erfindungsgemäss bevorzugten Ausführungsform umfasst die Mehr-Lumen-Verlängerung mindestens drei, vorzugsweise drei bis fünf Einzel-Lumen-Verlängerungen. Eine derartige Anzahl an Einzel-Lumen-Verlängerungen erlaubt eine optimale Ordnung der Einzel-Lumen-Verlängerungen im Einsatz und führt zum gewünschten Überblick für das Fachpersonal, beispielsweise in der Intensivmedizin.

Bei einer weiteren erfindungsgemäss bevorzugten Ausführungsform sind die Einzel-Lumen-Verlängerungen im Zwischenbereich flachbandartig angeordnet, derart, dass die zentralen longitudinalen Achsen der Einzel-Lumen-Verlängerungen in einer Ebene vorliegen. Auf Grund der flachbandartigen Anordnung, können Mehr-Lumen-Verlängerungen entsprechend übereinander als Stapel angeordnet sein.

Bei einer weiteren erfindungsgemäss bevorzugten Ausführungsform sind die Einzel-Lumen-Verlängerungen im Zwischenbereich bündelartig angeordnet, derart, dass jede Einzel-Lumen-Verlängerung an mindestens zwei benachbarten Einzel-Lumen-Verlängerungen befestigt ist. Dies bewirkt ebenfalls eine platzsparende Verteilung der Einzel-Lumen-Verlängerungen für den Transport, das Verpacken oder die Lagerung, wobei die bündelartige Anordnung eine optimale Packungsdichte erlaubt und so die gerichtete Ordnung zusätzlich unterstützt. Eine weitere bündelartige Anordnung kann derart gestaltet sein, dass die verschweissten oder verklebten Einzel-Lumen-Verlängerungen kreisartig einen begrenzenden zentralen Hohlraum ausbilden, in welchem ein flexibles, längliches Stützelement angeordnet sein kann.

Bei einer weiteren erfindungsgemäss bevorzugten Ausführungsform weisen die Einzel-Lumen-Verlängerungen einen identischen kreisrunden Querschnitt auf. Dies hat den Vorteil, dass eine einfache Herstellung der Einzel-Lumen-Verlängerungen möglich ist.

Bei einer weiteren erfindungsgemäss bevorzugten Ausführungsform besteht jeder der Einzel-Lumen-Verlängerungen aus einem Material, welches eine andere Farbe aufweist als sämtliche anderen Einzel-Lumen-Verlängerungen der Mehr-Lumen-Verlängerung. Diese farbliche Kennzeichnung erlaubt dem Anwender, wie beispielsweise dem Fachpersonal in der Intensivmedizin, den Überblick zu behalten, wie oben ausgeführt.

Bei einer weiteren erfindungsgemäss bevorzugten Ausführungsform ist das distale Kupplungselement als männlicher Luer-Lock Anschluss ausgestaltet. Entsprechende männliche Luer-Lock Anschlüsse sind kommerziell erhältlich und dienen als Kupplungselement für das Gegenstück, den weiblichen Luer-Lock Anschluss, welchem beispielsweise eine medizinische Quelle zugeordnet sein kann.

Bei einer weiteren erfindungsgemäss bevorzugten Ausführungsform ist das proximale Kupplungselement als weiblicher Luer-Lock Anschluss ausgestaltet. Entsprechende weibliche Luer-Lock Anschlüsse sind ebenfalls kommerziell erhältlich und dienen als Kupplungselement für das Gegenstück, den männlichen Luer-Lock Anschluss, welchem beispielsweise ein Katheter zugeordnet sein kann.

Die kommerziell erhältlichen Luer-Lock Anschlüsse, sei es männlich oder weiblich, sind üblicherweise aus Acrylnitril-Butadien-Styrol-Copolymerisat (ABS) gefertigt. Denkbar ist auch die Verwendung alternativer männlicher und weiblicher Anschlüsse, um die oben erläuterten Verbindungen zu gewährleisten.

Bei einer weiteren erfindungsgemäss bevorzugten Ausführungsform liegen die Mehr-Lumen-Verlängerungen in einer Verpackung steril vor.

Weitere Vorteile der vorliegenden Erfindung einer Mehr-Lumen-Verlängerung gehen aus der nachstehenden

Beschreibung von Ausführungsbeispielen hervor, welche anhand der Zeichnung erläutert werden.

Es zeigen rein schematisch:
- Fig. 1: in Ansicht eine erfindungsgemässe Mehr-Lumen-Verlängerung, wobei drei unterschiedlich gefärbte Einzel-Lumen-Verlängerungen flachbandartig angeordnet sind;
- Fig. 2: einen Querschnitt und einen Abschnitt des Zwischenbereichs durch die Mehr-Lumen-Verlängerung, gemäss Fig. 1 entlang der Linie II-II wobei die Einzel-Lumen-Verlängerungen unterschiedlich gefärbt sind;
- Fig. 3: im Querschnitt eine Mehr-Lumen-Verlängerung mit einer flachbandartigen Anordnung von fünf unterschiedlich gefärbten Einzel-Lumen-Verlängerungen im Zwischenbereich;
- Fig. 4: einen Querschnitt und einen Abschnitt des Zwischenbereichs einer Mehr-Lumen-Verlängerung mit einer bündelartigen Anordnung von drei unterschiedlich gefärbten Einzel-Lumen-Verlängerungen im Zwischenbereich;
- Fig. 5: im Querschnitt eine Mehr-Lumen-Verlängerung mit einer bündelartigen Anordnung von fünf unterschiedlich gefärbten Einzel-Lumen-Verlängerungen im Zwischenbereich;
- Fig. 6: im Querschnitt eine Mehr-Lumen-Verlängerung mit einer weiteren bündelartigen Anordnung von fünf unterschiedlich gefärbten Einzel-Lumen-Verlängerungen im Zwischenbereich mit einem ausgebildeten Hohlraum.

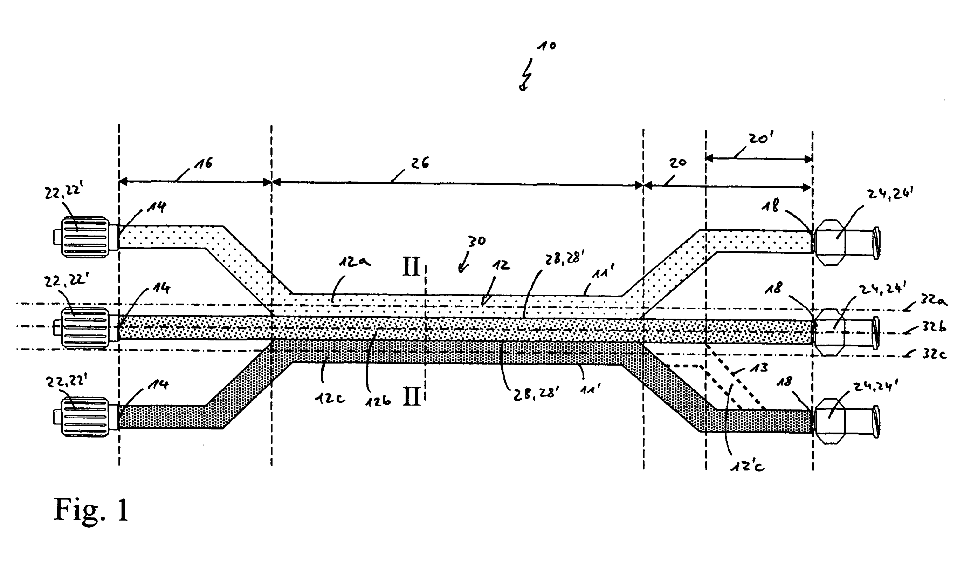

Die in Fig. 1 gezeigte erfindungsgemässe Ausführungsform einer Mehr-Lumen-Verlängerung 10 mit drei flexiblen, longitudinalen, unterschiedlich gefärbten, je einen Innenmantel 11 mit einem wenigstens annährend kreisrunden Querschnitt und je einen Aussenmantel 11' mit einem wenigstens annährend kreisrunden Querschnitt aufweisenden Einzel-Lumen-Verlängerungen 12 ist flachbandartig ausgebildet und jede dieser Einzel-Lumen-Verlängerungen 12a, 12b, 12c weist je einen an ein distales Ende 14 anschliessenden distalen Endbereich 16 und an je einen an ein proximales Ende 18 anschliessenden proximalen Endbereich 20 auf.

Dem distalen Ende 14 ist ein distales Kupplungselement 22, insbesondere ein männlicher Luer-Lock Anschluss 22' an betreffendem Endbereich 16 montiert. Mittels diesem männlichen Luer-Lock Anschluss 22' kann gegebenenfalls die betreffende Einzel-Lumen-Verlängerung 12 an eine medizinische Quelle (nicht gezeichnet), wie beispielsweise eine Infusionspumpe oder einen Infusionsbeutel, angeschlossen sein.

Dem proximalen Ende 18 ist ein proximales Kupplungselement 24, insbesondere ein weiblicher Luer-Lock Anschluss 24' an betreffendem Endbereich 20 montiert. Mittels diesem weiblichen Luer-Lock Anschluss 24' kann gegebenenfalls die betreffende Einzel-Lumen-Verlängerung 12 an einen Katheter (nicht gezeichnet) in Form eines Ballonkatheters oder eines nadelförmigen Katheters, auch als Zugang bezeichnet, angeschlossen sein.

Die drei Einzel-Lumen-Verlängerungen 12a, 12b, 12c der erfindungsgemässen Mehr-Lumen-Verlängerung 10 sind in einem zwischen dem distalen Endbereich 16 und dem proximalen Endbereich 20 liegenden Zwischenbereich 26 nebeneinander angeordnet und aneinander befestigt.

Mindestens im proximalen Endbereich 20 und distalen Endbereich 16 sind die Einzel-Lumen-Verlängerungen 12a, 12b, 12c voneinander getrennt, so dass ein grosser Freiheitsgrad in diesen Endbereichen 20, 16 gegeben ist und entsprechend ist der Anschluss von medizinischen Quellen und/oder von Kathetern gegebenenfalls einfacher handhabbar.

Bei Verwendung von mehr als zwei Einzel-Lumen-Verlängerungen 12 ist es auch möglich, dass die Endbereiche 16, 20 unterschiedlich lang ausgebildet sein können, um den Freiheitsgrad zu den medizinischen Quellen und/oder zu den Kathetern zu ermöglichen. In Fig. 1 wurde beispielhaft mit gestrichelten Linien 13 die Einzel-Lumen-Verlängerung 12c auf 12'c verlängert, wobei erkennbar wird, dass sich auch der entsprechende proximale Endbereich 20' neu ausbildet, im Vergleich zu den beiden benachbarten Einzel-Lumen-Verlängerungen 12a und 12b die den ursprünglichen proximalen Endbereich 20 beibehalten.

Bevorzugt ist die Befestigung 28 derart gestaltet, dass die Einzel-Lumen-Verlängerungen 12a, 12b, 12c entweder miteinander verschweisst oder verklebt sind.

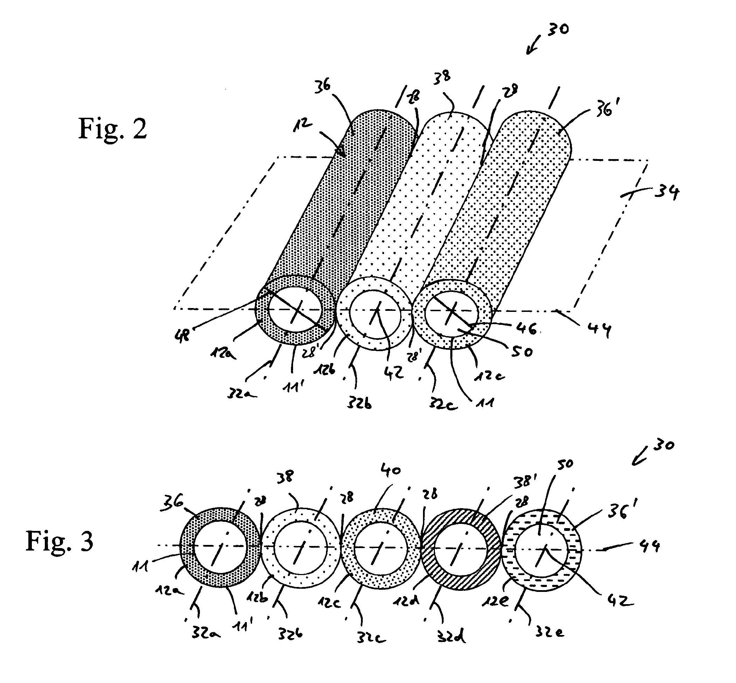

Die flachbandartige Anordnung 30 der drei Einzel-Lumen-Verlängerungen 12a, 12b, 12c gemäss Fig. 1 ist derart gestaltet, dass die zentralen longitudinalen Achsen 32a, 32b, 32c der Einzel-Lumen-Verlängerungen 12a, 12b, 12c gemeinsam in einer Ebene 34 verlaufen. Es wird in diesem Zusammenhang auf die Fig. 2 und 3 verwiesen, wo im Detail zwei Ausführungsformen der flachbandartigen Anordnung 30 der Mehr-Lumen-Verlängerung 10 erläutert werden.

Fig. 2 und 3 zeigen flachbandartige Anordnungen 30 der erfindungsgemässen Mehr-Lumen-Verlängerung 10 mit drei beziehungsweise fünf Einzel-Lumen-Verlängerungen 12. Die jeweils äussersten Einzel-Lumen-Verlängerungen 36, 36' sind mit den benachbarten innen liegenden Einzel-Lumen-Verlängerungen 38 bzw. 38' im Zwischenbereich 26 verbunden. Die innerste Einzel-Lumen-Verlängerung 40 ist mit den jeweils benachbarten innen liegenden Einzel-Lumen-Verlängerungen 38 bzw. 38' verbunden.

Die Aussenmäntel 11' von mindestens zwei benachbarten Einzel-Lumen-Verlängerungen 12a, 12b bilden im Zwischenbereich 26 mindestens abschnittsweise einen linienförmigen Berührungsabschnitt 28' (siehe Fig. 2) und die Verschweissung oder Verklebung liegt in diesem linienförmigen Berührungsabschnitt 28' als Befestigung 28 vor. Ausserhalb der Verschweissung oder Verklebung sind die Aussenmäntel 11' bei allen Ausführungsformen zueinander distanziert.

Es ist den Fig. 2 und 3 zu entnehmen, dass die Einzel-Lumen-Verlängerungen 12 einen inneren Durchmesser 46 und äusseren Durchmesser 48 aufweisen. Entsprechend ist die Einzel-Lumen-Verlängerung 12 beispielsweise ein schlauchförmiges Gebilde aus PVC mit einem kreisrunden Querschnitt 50. In bevorzugter Weise liegt der innere Durchmesser 46 bei 1.0 mm und der äussere Durchmesser 48 bei 2.1 mm. Ebenso ist es auch möglich Einzel-Lumen-Verlängerungen 12 mit anderen Dimensionen zu verwenden. Es sei an dieser Stelle erwähnt, dass der innere Durchmesser 46 den Durchmesser des Innenmantels 11 der Einzel-Lumen-Verlängerung definiert und der äussere Durchmesser 48 definiert den Durchmesser des Aussenmantels 11' der Einzel-Lumen-Verlängerungen und wird exemplarisch für alle Ausführungsformen in Fig. 2 gezeigt.

Eine weitere Möglichkeit die flachbandartige Anordnung 30 im Zwischenbereich 26 zu umschreiben besteht darin, dass in einer beliebigen Ebene 34, welche senkrecht zur longitudinalen Achse 32 der Mehr-Lumen-Verlängerung 10 verläuft, sämtliche Schnittpunkte 42 zwischen der Ebene 34 und den zentralen longitudinalen Achsen 32 der Einzel-Lumen-Verlängerungen 12 auf einer Geraden 44 liegen.

Der grundsätzliche Vorteil einer flachbandartigen Anordnung 30 liegt in der gerichteten Ordnung, so dass das Fachpersonal beispielsweise in der Intensivmedizin, schnell und einfach auf die relevanten Einzel-Lumen-Verlängerungen 12 Zugriff hat und so ein schnelles Anschliessen oder Trennen von medizinischen Quellen und/oder Kathetern möglich ist.

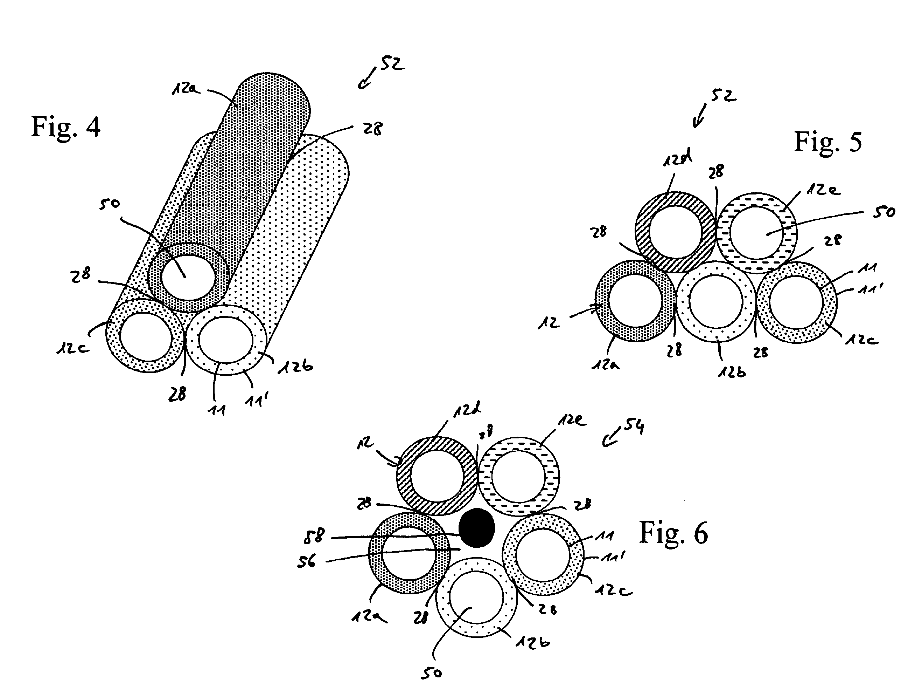

Die in Fig. 4 gezeigte Ausführungsform einer bündelartigen Anordnung 52 von drei unterschiedlich gefärbten Einzel-Lumen-Verlängerungen 12a, 12b, 12c ist derart, dass beispielsweise die Einzel-Lumen-Verlängerung 12a an den zwei benachbarten Einzel-Lumen-Verlängerungen 12b und 12c nebeneinander angeordnet ist und allesamt aneinander verschweisst oder verklebt sind.

Die in Fig. 5 gezeigte Ausführungsform der bündelartigen Anordnung 52 von fünf unterschiedlich gefärbten Einzel-Lumen-Verlängerungen 12a, 12b, 12c, 12d, 12e ist derart, dass die Einzel-Lumen-Verlängerungen 12a, 12b, 12c flachbandartig benachbart nebeneinander angeordnet sind und die zwei Einzel-Lumen-Verlängerungen 12d, 12e liegen auf der flachbandartigen Anordnung 30 versetzt benachbart an, so dass allesamt benachbart aneinander verschweisst oder verklebt sind.

Die bündelartige Anordnung 52 der Mehr-Lumen-Verlängerung 10 weist den Vorteil auf, dass die Einzel-Lumen-Verlängerungen 12 räumlich platzsparend verteilt sind, was wiederum eine sichere Zuordnung beim Einsatz in der Intensivmedizin ermöglicht, indem der Zeitaufwand für das Handling und die Verwechslungsgefahr im Einsatz erheblich minimiert wird.

Im Unterschied zu den Fig. 4 und 5 zeigt Fig. 6 eine weitere bündelartige Anordnung 54, bei der die fünf unterschiedlich gefärbten Einzel-Lumen-Verlängerungen 12a, 12b, 12c, 12d, 12e kreisartig angeordnet sind und dabei einen begrenzenden zentralen Hohlraum 56 ausbilden. Es besteht die Möglichkeit im zentralen Hohlraum 56 beispielsweise ein flexibles, längliches Stützelement 58 anzuordnen.

Es sei an dieser Stelle erwähnt, dass erfindungsgemäss die Mehr-Lumen-Verlängerung 10 mindestens zwei gefärbte bzw. mit einer Farbcodierung versehene Einzel-Lumen-Verlängerungen 12 aufweist.

In bevorzugter Weise werden, zur Bildung eines Vorrats, im Extrusionsverfahren flexible Einzel-Lumen-Verlängerungen 12 hergestellt, nebeneinander angeordnet und, wie oben erläutert, miteinander verschweisst oder verklebt. Von diesem Vorrat werden Abschnitte abgetrennt, welche mindestens zwei longitudinale, unterschiedliche Farbcodierungen aufweisende Einzel-Lumen-Verlängerungen 12 aufweisen. Vom proximalen Ende 18 her, werden senkrecht zur longitudinalen Achse 32 bewegend sukzessive die Einzel-Lumen-Verlängerungen 12 der gegenseitigen Befestigung 28 im proximalen Endbereich 20 aufgetrennt und an die proximalen Enden 18 der Einzel-Lumen-Verlängerungen 12 wird je ein proximales Kupplungselement 24 montiert. Vom distalen Ende 14 her, werden senkrecht zur longitudinalen Achse 32 bewegend sukzessive die Einzel-Lumen-Verlängerungen 12 der gegenseitigen Befestigung 28 im distalen Endbereich 16 aufgetrennt und an die distalen Enden 14 der Einzel-Lumen-Verlängerungen 12 wird je ein distales Kupplungselement 22 montiert.

Bei der Verwendung der Mehr-Lumen-Verlängerung 10 beispielsweise in der Intensivmedizin kann es durchaus vorkommen, dass auf Grund der örtlichen Situation die Einzel-Lumen-Verlängerungen 12 der Mehr-Lumen-Verlängerung 10 im jeweiligen Endbereich 16, 20 unterschiedlich lang von der gegenseitigen Befestigung aufgetrennt sind, so dass es möglich ist mit einem grossen Freiheitsgrad im jeweiligen Endbereich 16, 20 den Anschluss oder das Trennen von medizinischen Quellen und/oder Kathetern zu ermöglichen. Somit ergeben sich bei einem Einsatz von mehr als zwei Einzel-Lumen-Verlängerungen 12, je nach Situation, wie beispielhaft in Fig. 1 für die Einzel-Lumen-Verlängerung 12c angedeutet, unterschiedlich lang ausgebildete proximale Endbereiche 20 und 20'.

Es ist auch denkbar eine oder mehrere der Einzel-Lumen-Verlängerungen 12 der erfindungsgemässen Mehr-Lumen-Verlängerung 10 als Multi-Lumen-Verlängerung auszubilden. Durch entsprechende Einzel-Lumen-Verlängerung 12 als Multi-Lumen-Verlängerung, können beispielsweise zwei medizinische Quellen einem Patienten die Flüssigkeit zugeführt werden, ohne dabei den Zugang zu wechseln.

## Patentansprüche

1. Mehr-Lumen-Verlängerung zur medizinischen Anwendung umfassend mindestens zwei longitudinale, unterschiedliche Farbcodierungen, einen Innenmantel (11) und einen Aussenmantel (11') aufweisende Einzel-Lumen-Verlängerungen (12), welche je einen an ein distales Ende (14) anschliessenden distalen Endbereich (16) und je einen an ein proximales Ende (18) anschliessenden proximalen Endbereich (20) aufweisen, wobei dem distalen Ende (14) ein distales Kupplungselement (22) zugeordnet ist, die Einzel-Lumen-Verlängerungen (12) in einem zwischen dem distalen Endbereich (16) und dem proximalen Endbereich (20) liegenden Zwischenbereich (26) nebeneinander angeordnet und mindestens abschnittsweise aneinander befestigt sind und in den beiden Endbereichen (16, 20) die Einzel-Lumen-Verlängerungen voneinander getrennt sind, **dadurch gekennzeichnet, dass** der Innenmantel (11) und der Aussenmantel (11') der Einzel-Lumen-Verlängerungen (12) einen wenigstens annährend kreisrunden Querschnitt aufweisen und dem proximalen Ende (18) ein proximales Kupplungselement (24) zugeordnet ist.

2. Mehr-Lumen-Verlängerung nach Anspruch 1, **dadurch gekennzeichnet, dass** die Einzel-Lumen-Verlängerungen (12) im Zwischenbereich (26) ununterbrochen aneinander befestigt sind.

3. Mehr-Lumen-Verlängerung nach Anspruch 1 oder 2, **dadurch gekennzeichnet, dass** die Befestigung (28) zwischen den Einzel-Lumen-Verlängerungen (12) vom distalen Endbereich (16) und vom proximalen Endbereich (20) her lösbar ist.

4. Mehr-Lumen-Verlängerung nach einem der Ansprüche 1 bis 3, **dadurch gekennzeichnet, dass** die Einzel-Lumen-Verlängerungen (12) im Zwischenbereich (26) mindestens abschnittsweise verschweisst oder verklebt sind.

5. Mehr-Lumen-Verlängerung nach Anspruch 4, **dadurch gekennzeichnet, dass** die Aussenmäntel von mindestens zwei benachbarten Einzel-Lumen-Verlängerungen im Zwischenbereich mindestens abschnittsweise einen linienförmigen Berührungsabschnitt bilden und die Verschweissung oder Verklebung in diesem linienförmigen Berührungsabschnitt liegt.

6. Mehr-Lumen-Verlängerung nach einem der Ansprüche 1 bis 5, **gekennzeichnet durch** mindestens drei Einzel-Lumen-Verlängerungen (12).

7. Mehr-Lumen-Verlängerung nach Anspruch 6, **dadurch gekennzeichnet, dass** die Einzel-Lumen-Verlängerungen (12) im Zwischenbereich (26) flachbandartig nebeneinander angeordnet sind.

8. Mehr-Lumen-Verlängerung nach Anspruch 6, **dadurch gekennzeichnet, dass** die Einzel-Lumen-Verlängerungen (12) im Zwischenbereich (26) bündelartig angeordnet sind, derart, dass jede Einzel-Lumen-Verlängerung (12) an mindestens zwei benachbarten Einzel-Lumen-Verlängerungen (12) befestigt ist.

9. Mehr-Lumen-Verlängerung nach einem der Ansprüche 1 bis 8, **dadurch gekennzeichnet, dass** die Einzel-Lumen-Verlängerungen (12) einen, vorzugsweise zueinander identischen, kreisrunden Querschnitt (50) aufweisen.

10. Mehr-Lumen-Verlängerung nach einem der Ansprüche 1 bis 9, **dadurch gekennzeichnet, dass** jeder der Einzel-Lumen-Verlängerungen (12) aus einem Material besteht, welches eine andere Farbe aufweist, als sämtliche anderen Einzel-Lumen-Verlängerungen (12) der Mehr-Lumen-Verlängerung (10).

11. Mehr-Lumen-Verlängerung nach einem der Ansprüche 1 bis 10, **dadurch gekennzeichnet, dass** das distale Kupplungselement (22) als männlicher Luer-Lock Anschluss (22') ausgestaltet ist.

12. Mehr-Lumen-Verlängerung nach einem der Ansprüche 1 bis 11, **dadurch gekennzeichnet, dass** das proximale Kupplungselement (24) als weiblicher Luer-Lock Anschluss (24') ausgestaltet ist.

13. Mehr-Lumen-Verlängerung nach einem der Ansprüche 1 bis 12, gekennzeichnet, dass sie (10) in einer Verpackung steril vorliegen.

14. Verfahren zur Herstellung von Mehr-Lumen-Verlängerungen zur medizinischen Anwendung nach einem der Ansprüche 1 bis 13, bei dem von einem Vorrat Abschnitte abgetrennt werden, welche mindestens zwei longitudinale, unterschiedliche Farbcodierungen aufweisende Einzel-Lumen-Verlängerungen (12), welche nebeneinander angeordnet und aneinander befestigt sind, aufweisen, von einem proximalen Ende (18) der Abschnitte her die gegenseitige Befestigung (28) der Einzel-Lumen-Verlängerungen (12) in einem proximalen Endbereich (20) aufgetrennt und an die proximalen Enden (18) der Einzel-Lumen-Verlängerungen (12) je ein proximales Kupplungselement (24) montiert wird, und von einem distalen Ende (14) der Abschnitte her die gegenseitige Befestigung (28) der Einzel-Lumen-Verlängerungen (12) in einem distalen Endbereich (16) aufgetrennt und an die distalen Enden (14) der Einzel-Lumen-Verlängerungen (12) je ein distales Kupplungselement (22) montiert wird.

15. Verfahren zur Herstellung einer Mehr-Lumen-Verlängerung nach Anspruch 14, **dadurch gekennzeichnet, dass** zur Bildung des Vorrats die Einzel-Lumen-Verlängerungen (12) im Extrusionsverfahren hergestellt und miteinander verschweisst oder verklebt werden.
